# EUROPEAN PATENT APPLICATION

(11) **EP 2 166 108 A1**
(43) Date of publication of application: **24.03.2010**
(21) Application number: 09179065.9
(22) Date of filing: 05.08.2004
(51) Int. Cl.: C12P 17/04, C12P 7/40

(54) **Improved process for the production of mycophenolic acid**

(62) Divisional of application: 04398006.9
(71) Applicant: TECNIMEDE-SOCIEDADE TECNICO-MEDICINAL, S.A., 2685-338 Prior Velho (PT)
(72) Inventor: Castanhas Barbosa Gomes, Rui Jorge, 2640-066, Santo Isidoro (PT); Vazão Mano Clemente, João José, 2710-695, Sintra (PT); Almeida Bezerra Fernandes Thomaz, Mónica Maria, 2765, Estoril (PT); Barbosa Pereira Da Cunha, António Eduardo Pio, 2600-000, Castanheira do Ribatejo (PT)
(74) Representative: Viering, Jentschura & Partner

(57) **Abstract**

According to the present invention mycophenolic acid (MPA) is produced in submerged fermentation, by using mutant of *Penicillium brevicompactum* ATCC16024 (IMI 17456) in aerobic conditions. The process of the invention consists in two or more fermentations in sequence, being one or more seed fermentations and one production fermentation. In the production fermentation a shift in temperature and/or pH is made at certain biomass concentration and accordingly MPA concentrations of 3.4g/L in the fed-batch reactor and 2.6g/L in the batch reactor were attained. The inoculum percentage was optimized to maximize the final MPA concentration.

Additionally, the process according to the invention follows a biomass separation method, which comprises a chromatographic step using an adsorption resin. After the elution using an organic solvent, a precipitation step is performed using chilled water at low pH, the precipitate is then centrifuged and dried in an oven. The MPA purity attained is approximately 98%.

The process according to the present invention has the advantages of high productivity, of using simple processes and was designed baring in mind the scalability of the entire process to a full production scale. Pilot scale was used to test the scale-up parameters. A fermentation with working volume of 300L was used.

## Description

### SUMMARY OF THE INVENTION

The present invention relates to an improved process for the production of mycophenolic acid (MPA). This process consists in two or more fermentations in sequence, being one or more seed fermentations and one production fermentation. Furthermore, the process of the invention follows a biomass separation method, which comprises a chromatographic step using an adsorption resin.

### PRIOR ART

Mycophenolic acid (MPA), ((E)-6-(1,3-di-hydro-4-hydroxy-6-metoxy-7-methyl-3-oxo-5-isobenzofuranyl)-4-methyl-4-hexenoic) acid) was obtained for the first time using a *Penicillium* (B. Rosio, Riv. Igiene Sanita Pub. Ann., 7, 825-869, 1896 or later - Oxford, P.W. Clutterbuck et al, Biochem.J. 26, 1442-1458 (1932)).

Nowadays, mycophenolic acid is used in esterified form, the morpholine ethyl ester, (2-(4-morpholynil)-ethyl ester) of the ((E)-6- (1,3-di-hydro-4-hydroxy-6-metoxy-7-methyl-3-oxo-5-isobenzofuranyl)-4-methyl-4-hexenoic acid), that acts as a pro-drug of the mycophenolic acid. This drug is used to prevent rejection of transplanted tissues or organs; it also has a potential use in the treatment of rheumatoid arthritis and psoriasis.

Although some attempts have been made to perform a total chemical synthesis of mycophenolic acid (e.g.: Danheiser Rick L et al, J.Am.Chem.Soc., 1986, 108, pp 806-810; Patterson J.W. et al, J.Chem.Soc.Chem.Commun., Vol. 53 n°9, 1997, pp 3395-3400 or Covarrubias-Zúñiga et al, Tetrahedron Lett. 39 (1998) 2880-2882) they have limited industrial applicability. For this reason mycophenolic acid has been produced industrially by aerobical fermentation processes using species of Penicillium.

The first patent for production of mycophenolic acid (GB 1157099 (1966) - Imperial Chemical Industries Ltd.) discloses a process for the production of MPA through a fermentation process using *Penicillium brevicompactum, Penicillium stoloniferum* and *Penicillium viridicatum.* The yields attained according to the claimed process vary between 14mg/L to 226mg/L of mycophenolic acid in the fermentation broth.

Patent GB 1158387 (1967) - Imperial Chemical Industries Ltd. - discloses a process for the production of mycophenolic acid using a similar fermentation process to that of GB 1157099 but the yields of MPA attained were in the order of 363mg/L with a purity of 77% or 70mg/L of MPA with a purity of 94.94%.

Patent GB 1593208 (1978) - Lilly & Co Elli - discloses an improved process for obtaining mycophenolic acid using a new mutant of the species *Penicillium.*

Patent US 4452891 (1981) - Ajinomoto KK - discloses an improved process for producing mycophenolic acid using a new mutant of the species *Penicillium.* This patent covers essentially the process for obtaining high producing mutants. MPA concentrations of 2.4g/L (in shaken flasks) and 3.6g/L (in non agitated flasks) were obtained. However, the small scale of the presented examples and the fermentation processes used do not favour the scalability of this processes to industrial production.

More recently the international patent application WO 01/64931 (2000) - Biocon India Ltd. - claims a solid state fermentation process for obtaining mycophenolic acid. Although it is not stated which massic productivity is obtained, it is still a solid state fermentation process which takes a very intensive work, is subject to extensive manipulation and problems with homogeneity are more disposed to occur.

Regarding the purification process, it involves many steps, like solvent extraction, concentration, crystallization, dissolution in a solvent, treatment with adsorbers, filtration, distillation and others. Industrially, this involves handling of great quantities of solvents, which can be problematic and costly both in terms of the solvents themselves and in terms of the equipment and plant requirements.

### DETAILED DESCRIPTION OF THE INVENTION

The MPA production by submerged fermentation is known in the art. The process yield in MPA using a wild type strain is normally quite low compared with a mutant resistant to polyen antibiotics.

Surprisingly, the process according to the present invention lead to high massic productivity, when compared to the processes described in the prior art, by using simple processes and taking into account the scalability of the entire process to a full production scale. Pilot scale was used to test the scale-up parameters, using a fermentation working volume of 300L.

According to the invention, the preparation of mycophenolic acid comprises two or more fermentations in sequence and a chromatographic step using an adsorption resin.

### FERMENTATION

According to the invention, the isolate used is a mutant that reveals increased MPA productivity over the original strain. Additionally, according to the present invention, the desired MPA concentration is attained at an early stage, which simplifies the purification processes, as less contaminants of difficult separation are produced. Also, according to the invention, a high inoculum concentration and a shift in temperature and/or pH at a certain biomass value reduces the fermentation time by increasing the rate of MPA production, as well as increasing the final concentration of MPA. Multi-stage fermentations are used to build-up the necessary inoculum volume to inoculate the production fermenter.

According to the present invention, the fermentation is a multi-stage fermentation in which the inoculum fermentations are run until the end of the base demand (alkali demand) and until the inoculum volume is within the range of 2% to 10%. The production fermentation is run from 190h to 260h or until MPA concentration stabilises, being the inoculum volume in the range of 10% to 30%.

The process according to the present invention, wherein the productivity of MPA is substantially increased and wherein a submerged culture and a mutant of *Penicillium brevicompactum* ATCC16024 (IMI 17456) resistant to clofibrate is used, is **characterized in that:**
- a carbon source in high concentrations, e.g. glycerol, glucose, sucrose, soybean oil or molasses, is used;
- a high *inoculum* concentration for the production fermenter (10% to 30% in biomass) is used;
- the inoculation for the production fermenter is made dependent on the change of metabolism derived from the depletion of the carbon source, which can be determined by the halt of base consumption;
- the production fermenter is run at pH values of 4.0 to 7.0 and at a temperature of 23°C to 28°C during the vegetative growth phase (up to biomass levels of 20% (w/w) wet weight to 35% (w/w) wet weight);
- mass sporulation is induced as the biomass reaches 20% (w/w) to 35% (w/w) with an increase of temperature of up to 5°C and an increase of pH up to 4 pH units;
- the process is run from 190h to 260h or until the MPA concentration stabilises.

The pre-inoculum is prepared from a slant containing the *Penicillium brevicompactum* strain and transferred to a liquid medium (Tabela 2).

**Table 1- Pre-inoculum Medium composition**

| | **Amounts for 1L medium** |
|---|---|
| Salts solution | 1.0 ml |
| Glucose | 50 to 150 g |
| Glycine | 5 to 20 g |
| L- Methionine | 0.5 g |
| KH₂PO₄ | 3 g |
| MgSO₄.7H₂O | 1 g |
| Antifoam | 1 g |
| Water | to 1 L |

**Table 2- Medium composition**

| | **Amounts for 1L medium** |
|---|---|
| Salts solution | 1.0 ml |
| Glycerol | 50 to 250 g |
| Glycine | 5 to 20 g |
| L- Methionine | 0.5 g |
| KH₂PO₄ | 3 g |
| MgSO₄.7H₂O | 1 g |
| Antifoam | 1 g |
| Water | to 1 L |

Thus, the pre-inoculum is incubated in an orbital shaker at 27°C and at 150 rpm during 72h to 96h, being the seed fermenter inoculated with 2% to 10% of the fermenter volume.

This fermenter is run at 23°C to 28°C, with aeration and stirring speed able to maintain pO₂ over 30%; being the pH kept constant at 4.0 to 7.0 by means of addition of NaOH 3M.

This fermentation runs for 72h to 96h, until the end of alkali demand. At this time, biomass should be between 10% (w/w) to 30% (w/w).

The production fermenter is inoculated with 10% to 30% of its volume. The production fermenter runs initially at 23°C to 28°C and at an aeration rate and stirring speed able do maintain pO₂ over 30%; the pH is kept at 4.0 to 7.0 by means of addition of NaOH 3M. When the biomass reaches 20% (w/w) to 35% (w/w) the temperature is increased up to 5°C and the pH is increased up to 4.0 pH units.

In case a batch process is used, the fermentation runs for 190h to 260h, or until MPA concentration stops increasing.

In the case of the fed-batch system, the carbon source is fed automatically whenever the alkali consumption ceases and the pH starts to rise. This way, the carbon source is kept at a minimum concentration and pH is controlled by substrate. The end of the fermentation is attained 180h after the shift of pH values and temperature, or when the MPA concentration stabilises.

### Example 1

The different fermentation modes and strategies of pH and temperature shifts are illustrated in table 3.

**Table 3 - Comparison between different strategies**

| **Experiment No.** | **1** | **2** | **3** |
|---|---|---|---|
| Strategies | A | B | C |

| **Conditions** | | | |
|---|---|---|---|
| Fermentation volume | 20 L | 20 L | 20 L |
| Airflow | 20 L/min | 20 L/min 20 L/min | 20 L/min |
| Agitation | 300-800 rpm | 300-800 rpm | 300-800 rpm |
| Temperature | Constant | With variation | With variation |
| pH | Constant | With variation | With variation |
| | | | |

| **Productivity** | | | |
|---|---|---|---|
| Culture time | <200 h | <200 h | >300 h |
| MPA concentration | 1.2 g/L | 2.6 g/L | 3.4 g/L |
| | | | |
| **Type of fermentation** | Batch | Batch | Fed-batch |

| | | | |
|---|---|---|---|
| A - Batch fermentation with constant pH and temperature B - Batch fermentation with shifts of pH and temperature C - Fed-batch fermentation with shifts of pH and temperature | | | |

The data showed in table 3, lead to the conclusion that the experiment performed at constant temperature and pH values resulted much less productive than those with variations in pH and temperature. The experiment carried out in fed-batch fermentation resulted in a highest concentration, although, the time necessary to reach that value was significantly higher.

### Example 2

The relevance of the inoculum biomass is stated in table 4.

**Table 4 - Comparison between different inoculum concentrations**

| **Experiment No.** | **2** | **5** |
|---|---|---|
| Inoculum biomass % (w/w) | 19 | 12 |
| [MPA] mg/L | 2354 | 1890 |
| Age (h) | 216 | 240 |
| | | |
| [MPA] mg/L at the age of 190h | 2200 | 1600 |

The percentage of the biomass - % of (w/w) - plays an important role in the final MPA concentration, specially, when concentrations over the same period of time are compared.

### PURIFICATION

According to the present invention, the purification is greatly simplified by using the right resin at a convenient load, and integrating with a fermentation strategy that prevents the formation and/or accumulation of hard to separate contaminants. Thus, in the first stage of MPA purification, an adsorption column is used instead of the normally processes of solvent extraction or counter-extraction. There is no evidence in the prior art of a process in which an adsorption column is used in such an early stage of the MPA purification.

The MPA purification process from fermentation starts by adjusting the pH of the fermentation broth to 7.0, after which a suitable operation of biomass removal is required, such as centrifugation or filtration, followed by an adsorption chromatography step. After the elution of the chromatographic column with an organic solvent such as methanol, follows a precipitation step using chilled water at low pH, e.g. 2.0. The precipitate is then washed with purified water at low pH and dried in an oven to obtain a white powder with an MPA content of 98%.

In the step of adsorption chromatography, the supernatant passes through the chromatographic column, containing the adsorption resin at the appropriate pH, at a flow rate of less than two column volumes per hour, being then the column washed with at least one column volume of NaCl 0.2 M at pH 7.0 at a flow rate not greater than 1 column volume per hour followed by a methanol solution at the same flow rate. The elution step is performed with 100% of an organic solvent, e.g. methanol, at a flow rate of less than one column volume per hour.

The eluted fractions containing MPA are further processed by mixing together 3 volumes of purified water at low pH and temperature, e.g. pH 2.0 and 4°C, to 1 volume of eluate and allowing the solution to precipitate overnight at low temperature. The resulting precipitate is then separated by a suitable mean, such as centrifugation or filtration and then washed with the same amount of purified water at low pH and temperature. The resulting precipitate is then dried in a vacuum oven at a temperature of 65°C to constant weight. The resulting precipitate is of white colour with a HPLC purity of at least 98%. The yield of the purification process is approximately 80%.

### Example 3

A volume of 160 L of fermentation supernatant containing 1 g/L of MPA was used to charge a column containing the adsorption resin. From the elution with methanol, a 14 L MPA rich pool was collected. 42 L of purified water at 4°C and pH 2.0 were added to the 14 L pool, mixed together and the pH was again adjusted to 2.0. The solution was then kept overnight at 4°C. Subsequently, the mixture was centrifuged at 4000 g and the pellet was washed with the same volume of chilled water at pH 2.0. The obtained solid was dried in a vacuum oven at 65°C to constant weight. 120g of a white powder were obtained and identified as MPA with a HPLC purity in excess of 98%.

## Claims

1. An improved process for the production of mycophenolic acid in a submerged *Penicillium brevicompactum* ATCC16024 (IMI 17456) mutant culture **characterised in that** it comprises the following consecutive steps:
a. having two or more fermentation stages;
b. inoculating the production fermentation from the seed fermentation;
c. using a carbon source with an initial concentration of 5 to 25%;
d. running the seed fermenter from 72 to 96 hours;
e. running the production fermenter from 190 to 260 hours;
f. shifting the temperature and/or pH during the production fermentation;
g. centrifugation or filtration to separate the biomass;
h. use of an adsorption resin after clarification;
i. elution of the resin using a solvent;
j. a precipitation step following elution;
k. centrifugation or filtration to remove the supernatant;
l. washing the precipitate;
m. drying the precipitate to obtain the dried MPA crystals.

2. The process according to claim 1, wherein the carbon source is sucrose, glucose, glycerol, soybean oil or molasses.

3. The process according to claim 1, **characterised in that** it runs in batch or carbon source fed-batch mode.

4. The process according to claim 1, wherein the last of the multi-stage fermentation steps is used for production while the others are used for vegetative growth.

5. The process according to claim 1, wherein the seed fermentations are:
a. inoculated with a volume in the range of 2% to 10%;
b. run at temperatures between 23°C and 28°C and pH between 4.0 and 7.0;
c. run until the end of the alkali demand.

6. The process according to claim 1, wherein the inoculation of the production fermenter is in the range of 10%(w/w) to 30%(w/w) in biomass.

7. The process according to claim 1, wherein the production fermentation runs from 190h to 260h or until the MPA concentration stops increasing.

8. The process according to claim 1, wherein in the production fermenter:
a. the temperature is shifted when the biomass is in the range of 20%(w/w) to 35%(w/w);
b. the pH is shifted when the biomass is in the range of 20%(w/w) to 35%(w/w);
c. a shift in the pH is performed by increasing it up to 4.0 pH units;
d. a shift in the temperature is performed by increasing it up to 5°C.

9. The process according to claim 1, wherein the biomass separation is followed by a step of adsorption to a resin.
